# EUROPEAN PATENT APPLICATION

(11) **EP 2 711 001 A2**
(43) Date of publication of application: **26.03.2014**
(21) Application number: 13182356.9
(22) Date of filing: 13.11.2009
(51) Int. Cl.: A61K 9/14, C07F 9/10, A61K 47/24, A23L 1/30, A23D 7/01, A23D 9/013, A61K 8/37, A61K 8/55, A61K 8/73, A61Q 17/00, A61K 9/127, A61K 8/04

(54) **Organogel compositions and processes for producing**

(30) Priority: 14.11.2008 US 114510 P
(62) Divisional of application: 09826843.6
(71) Applicant: Archer-Daniels-Midland Company, Decatur, Illinois 62526 (US)
(72) Inventor: Baseeth, Shireen S., IL, 62526 Decatur (US); Sebree, Bruce R., IL, 62501 Oakley (US)
(74) Representative: von Kreisler Selting Werner

(57) **Abstract**

The present invention is directed towards organogel compositions comprising a phospholipid composition. Processes for producing such organogel compositions are further disclosed. The present invention is also directed towards uses of the novel organogel compositions in foods or beverages, cosmetics, personal care products, as a drug delivery vehicle or as a carrier of any desired compound.

## Description

### TECHNICAL FIELD

The present invention relates generally to organogels. The present disclosure is directed to compositions comprising a phospholipid composition, an organic solvent, a bio-based natural polymer and a polar solvent. The present disclosure is also directed to methods for the preparation and use of the composition comprising the phospholipid composition, the organic solvent, the bio-based natural polymer and the polar solvent.

### BACKGROUND ART

Liquid crystalline structures are generally well ordered structures that can hold a large number of active ingredients, yet restrict the diffusion of the active ingredients to facilitate a controlled release of the active ingredients. However, some of the components used to create these cubic crystalline phases can be difficult to incorporate into such phases. For instance, monoglycerides have some undesirable physical characteristics such as a high melting point that makes the monoglycerides pastes or waxy solids at room temperature. Further, the equilibration time required to form the monoglycerides into such structures may be several hours or days since the diffusion of water through the solid monoglycerides is delayed.

Another problem is that the processes used to form the cubic, liquid crystalline phases are cumbersome since such processes require longer holding times, higher manufacturing temperatures, and high shear processes that are not economically or commercially viable.

Lecithin organogels are clear, thermodynamically stable, vicsoelastic and biocompatible jelly-like phases typically composed of hydrated, purified phospholipids, an organic liquid and a gelating agent. Typically, the purified phospholipids used contain at least 80-95% phosphatidylcholine content to prepare the organogel. A limitation of earlier organogel formation needs the use of very highly pure lecithin that is expensive and not easily obtained. The synthetic polymer, pluronic, has been used in lecithin organogels, The amount of pluronics typically used is between about 30-40%. However, pluronics are non-ionic triblock copolymers which may be characterized as a skin irritant, are not bio-based, not allowed in food systems and are not inexpensive compounds.

### DISCLOSURE OF INVENTION

The present invention overcomes the obstacles of the prior art and discloses a more commercially viable method to make cubic, liquid crystalline phases at ambient temperature without the input of high energy, with a low equilibration time in minutes or a few hours. The phospholipid organogels disclosed herein are highly ordered liquid crystalline structures are unique and generally are high-viscosity solid like gels that have the ability to carry a large amount of a compound such as an active ingredient. Such structured phospholipid organogels are thermo-reversible.

In one embodiment, a composition comprises a phospholipid composition, an organic solvent, a bio-based natural polymer and a polar solvent.

In another embodiment, a process for producing a product comprises mixing an organic solvent with a phospholipid composition, thus producing an organic phase; dispersing a bio-based natural polymer in a polar solvent, thus producing a polar phase; and mixing the organic phase with the polar phase.

In an additional embodiment, a composition comprises a phospholipid composition, an organic solvent, a xanthan gum and a polar solvent. The phospholipid composition, the organic solvent, the xanthan gum and the polar solvent are present in such amounts and processed such that the composition takes the form of a clear, thermodynamically stable, viscoelastic jelly-like phase.

In another embodiment, a thermo-reversible, structured phospholipid organogel composition comprises a phospholipid composition, an organic solvent, a water soluble polymer, and a polar solvent.

A further embodiment includes a process for producing a product, the process comprising mixing a organic solvent with a phospholipid composition, thus producing an organic phase; dispersing a water soluble polymer in a polar solvent, thus producing a polar phase; and mixing the organic phase with the polar phase.

An additional embodiment includes a method of loading a thermo-reversible, structured phospholipid organogel, the method comprising melting the thermo-reversible, structured phospholipid organogel, mixing a compound with the melted thermo-reversible, structured phospholipid organogel, and cooling the thermo-reversible, structured phospholipid organogel to a temperature below the melting point such that the organogel reforms to the shape of a gel.

### BRIEF DESCRIPTION OF THE DRAWINGS

Figure 1 is a representative viscosity profile of one embodiment of a lecithin organogel of the present invention.

Figure 2 shows the Small Angle X-ray Scattering for one embodiment of a lecithin organogel of the present invention.

Figures 3A and 3B illustrate viscosity profiles of embodiments of lecithin organogels including active ingredients of the present invention.

Figure 4 shows the Small Angle X-ray Scattering for one embodiment of a lecithin organogel of the present invention.

### MODES FOR CARRYING OUT THE INVENTION

In one embodiment, the present invention is directed towards processes for producing lecithin organogels, as well as the organogels produced there from.

In another embodiment, the present invention includes a composition comprising a phospholipid composition, an organic solvent, a bio-based natural polymer and a polar solvent.

In yet a further embodiment, the composition takes the form of a clear, thermodynamically stable, viscoelastic jelly-like phase. This may be accomplished by placing the phospholipid composition, the organic solvent, the bio-based natural polymer and the polar solvent in such amounts in the composition and processing the composition in such a manner to produce such a phase.

Lecithin organogels have a range of applications in cosmetics and personal care products, as well as utility in transdermal drug delivery systems for transporting actives through membranes. The ability of the purified phospholipids to be a good penetrant, solubilizer and its film forming properties make the purified phospholipids a good composition for bioactive transport applications. Topical applications of these organogels benefit from outstanding miniaturization, skin barrier strengthening and the uniform delivery of active substances.

In a further embodiment, the composition may be configured as a topical agent or cosmetic. In this embodiment, the composition may further comprise a compound selected from the group consisting of green tea extract, a fragrance, ascorbic acid, potassium sorbate, citric acid, natural polar antioxidants, tocopherols, sterols or phytosterols, saw palmetto, caffeine, sea weed extract, grape-seed extract, rosemary extract, almond oil, lavender oil, peppermint oil, bromelain, capsaicin, benzalkaonium chloride, triclosan, para-chloro-meta xylenol (PCMX), hyalauronic acid, emulsifiers or combinations of any thereof. In other embodiments, the organogels of the present invention may be used to solubilize polar, non-polar and/or amphilic guest molecules. In another embodiment, the organogels of the present invention may be used to solubilize or carry enzymes.

In still an additional embodiment, the composition may be configured a pharmaceutical delivery composition. In such embodiment, the composition may further comprise a compound selected from the group consisting of an anesthetic, a nonsteroidal anti-inflammatory drug, a muscle relaxant, a steroid, a hormone, an analgesic, an antiemetic, a cardiovascular agent, an antithyroid drug, a macromolecule, a neuropathy drug, a sanitizer, a disinfectant or combinations of any thereof.

In another embodiment, the composition may be used in a food product. In such embodiments, non-limiting uses of the composition include, without limitation: a structuring agent for providing or enhancing structure in foods such as, for example, in spreads, mayonnaise, dressings, shortenings, fluid oils, fillings, icings and frostings; an emulsifier that can be used to carry active ingredients or enzymes such as in baking applications; a film forming composition that can hold active ingredients; a coating or seasoning on a food that could hold spices or seasonings; a film-forming composition that could be used as a release agent; a beverage emulsion; or as a carrier for delivering nutritional or bio-active compounds.

In one embodiment, the phospholipid composition comprises lecithin produced by various processes. Lecithins suitable for use in the disclosed compositions and methods include, but are not limited to, crude filtered lecithin, standardized-fluid lecithins, de-oiled lecithin, chemically and/or enzymatically modified lecithins, alcohol fractionated lecithins, chromatagraphicly purified lecithins, purified lecithins, and blends of any thereof. A crude filtered lecithin having an HLB value of approximately 4.0 may be used. Standardized lecithin including additives having HLB values ranging from 10.0 to 24.0, which results in lecithin compositions having HLB values of 7.0 to 10.0 may be used. Any lecithin or combinations of lecithins are suitable for use in the disclosed compositions and methods regardless of the initial HLB value of the lecithin.

In another embodiment, the phospholipid composition comprises any purity. In various embodiments, the phospholipid composition has less than 90% phosphatides, has less than 30% phosphatidyl choline, has between 10-95% phosphatidyl choline content, or combinations of any thereof. The use of a lecithin having less than 90% phosphatides or less than 30% phosphatidyl choline is beneficial since such a composition is more economical to produce than using a lecithin composition having greater than 90% phosphatides or greater than 30% phosphatidyl choline.

In one embodiment, the lecithin comprises ULTRALEC P brand deoiled lecithin available from Archer Daniels Midland Company, Decatur, Illinois. Deoiled lecithin is typically in dry form of a powder, fine granule or a granule, and comprises a minimum of 97.0% acetone insolubles as determined by AOCS Ja 4-46, a maximum of 1.0% moisture as determined by AOCS Ja 2b-87, a maximum of 0.05% of hexane insolubles as determined by AOCS Ja 3-87, and an effective HLB value of approximately 7.

In another embodiment, the lecithin comprises YEKLIN SS brand lecithin available from Archer Daniels Midland Company, Decatur, Illinois. This lecithin is a light amber liquid and comprises a minimum of 62.00% acetone insolubles as determined by AOCS Ja 4-46, has a maximum acid value of 30.00 mg KOH/g as determined by AOCS Ja 6-55, a maximum of 1.0% moisture as determined by AOCS Ja 2b-87, a maximum color (Gardner, as is) of 14.00 as determined by AOCS Ja 9-87, a maximum of 0.05% hexane insolubles as determined by AOCS Ja 3-87, a maximum viscosity of 100 stokes at 77 degrees as determined by AOCS Ja-87 and an effective HLB value of approximately 4.

In a further embodiment, the lecithin comprises THERMOLEC WFC brand hydroxylated soy lecithin available from Archer Daniels Midland Company, Decatur, Illinois. This lecithin is a translucent liquid and comprises a minimum of 60.00% acetone insolubles as determined by AOCS Ja 4-46, has a maximum acid value of 30.00 mg KOH/g as determined by AOCS Ja 6-55, a maximum of 1.0% moisture as determined by AOCS Ja 2b-87, a maximum color (Gardner, as is) of 13.00 as determined by AOCS Ja 9-87, a maximum of 0.05% hexane insolubles as determined by AOCS Ja 3-87, a maximum peroxide value of 10.0 as determined by AOCS Ja 8-87 and a maximum viscosity of 100 stokes at 77 degrees as determined by AOCS Ja 11-87.

In an additional embodiment, the lecithin comprises THERMOLEC 200 brand soy lecithin available from Archer-Daniels-Midland Company, Decatur, Illinois. This lecithin is a translucent liquid and comprises a minimum of 62.00% acetone insolubles as determined by AOCS Ja 4-46, has a maximum acid value of 30.00 mg KOH/g as determined by AOCS Ja 6-55, a maximum of 0.8% moisture as determined by AOCS Ja 2b-87, a maximum color (Gardner, as is) of 14.00 as determined by AOCS Ja 9-87, a maximum of 0.05% hexane insolubles as determined by AOCS Ja 3-87, a maximum peroxide value of 5.0 as determined by AOCS Ja 8-87, a maximum viscosity of 75 stokes at 77 degrees as determined by AOCS Ja 11-87 and an effective HLB value of approximately 7.

In a further embodiment, the biobased natural polymer comprises xanthan gum, gellan gum, cellulose and modified cellulose products, starch, chitin, carrageenan, gum arabic, an alginate, gum acacia, guar gum, agar, gelatin, locus bean gum, inulin, maltodextrin, pectin, beta glucans or combinations of any thereof. In an additional embodiment, the biobased natural polymer may be present in a concentration of between 0.5-1.0%. In other embodiments, water soluble polymers that are synthetic or natural could be used.

In one embodiment, the organic solvent comprises isopropyl myristate, ethyl laureate, ethyl myristate, isopropyl palmitate, cyclopentane, cyclooctane, trans-decalin, trans-pinane, n-pentane, n-hexane, n-hexadecane, tripropylamine, 1,7-octadiene, butyl laurate, cyclododecane, dibutyl ether, isooctane, n-octane, tributylamine, triisobutylamine, mineral oil, vegetable oil such as triglyceride and/or diglyceride oils, a polyol esters, monoglycerides, diglycerides, fatty acid esters, or combinations of any thereof.

In one embodiment, the polar solvent comprises water, glycerol, ethylene glycol, propylene glycol, formamide, isosorbide, isosorbide derivatives, sorbitol, erythritol, other polyhydric alcohols or combinations of any thereof.

In one embodiment, the compositions described herein are bio-based. Bio-based content of a product may be verified by ASTM International Radioisotope Standard Method D 6866. ASTM International Radioisotope Standard Method D 6866 determines bio-based content of a material based on the amount of bio-based carbon in the material or product as a percent of the weight (mass) of the total organic carbon in the material or product. Bio-derived and bio-based products will have a carbon isotope ratio characteristic of a biologically derived composition.

In an additional embodiment, each of the components of the compositions of the present invention are edible and/or approved for use in foods.

The invention is further explained by use of the following exemplary embodiments.

### Example 1.

An organic phase was prepared by adding YELKIN SS brand lecithin, available from Archer-Daniels-Midland Company of Decatur, Illinois, at 80% concentration by weight to 16% isopropyl myristate and dissolving the lecithin in the isopropyl myristate with constant stirring at room temperature.

A polar phase was prepared by dispersing NOVAXAN D brand xanthan gum, a water dispersible transparent xanthan gum, available from Archer-Daniels-Midland Company of Decatur, IL, at 0.6-1.0% in distilled water at room temperature.

The polar phase was slowly introduced into the organic phase under constant stirring at a concentration of 4% at room temperature. At this point, the lecithin organic phase spontaneously changed from a Newtonian fluid to a viscous gel phase, also referred to as the lecithin organogel. Upon heating, the lecithin organogel became fluid and self-assembled back into the lecithin organogel upon cooling, indicating the thermo-reversible property of the lecithin organogel.

### Example 2.

An organic phase was prepared by adding THERMOLEC WFC brand lecithin, an acetylated and hydroxylated heat resistant lecithin, available from Archer-Daniels-Midland Company of Decatur, IL, at 85% concentration by weight to isopropyl myristate at 11% by weight concentration, and dissolving the lecithin in the isopropyl myristate with constant stirring at room temperature.

A polar phase was prepared by dispersing NOVAXAN D brand xanthan gum, a water dispersible transparent xanthan gum, available from Archer-Daniels-Midland Company of Decatur, IL, at 0.6-1.0% in distilled water at room temperature.

The polar phase was slowly introduced into the organic phase under constant stirring at a concentration of 4% at room temperature. At this point, the lecithin organic phase spontaneously changed from a Newtonian fluid to a viscous gel phase, also referred to as the lecithin organogel. Upon heating, the lecithin organogel became fluid and self-assembled back into the lecithin organogel upon cooling, indicating the thermo-reversible property of the lecithin organogel.

### Example 3.

An organic phase was prepared by adding THERMOLEC 200 brand lecithin, an acetylated heat resistant lecithin, available from Archer-Daniels-Midland Company of Decatur, IL, at 80% concentration by weight to isopropyl myristate and dissolving the lecithin in the isopropyl myristate with constant stirring at room temperature.

A polar phase was prepared by dispersing NOVAXAN D brand xanthan gum, a water dispersible transparent xanthan gum, available from Archer-Daniels-Midland Company of Decatur, IL, at 0.6-1.0% in distilled water at room temperature.

The polar phase was slowly introduced into the organic phase under constant stirring at a concentration of 4% at room temperature. At this point, the lecithin organic phase spontaneously changed from a Newtonian fluid to a viscous gel phase, also referred to as the lecithin organogel, Upon heating, the lecithin organogel became fluid and self-assembled back into the lecithin organogel upon cooling, indicating the thermo-reversible property of the lecithin organogel.

### Example 4.

An organic phase was prepared by adding ULTRALEC P brand lecithin, a deoiled lecithin, available from Archer-Daniels-Midland Company of Decatur, IL, at 80% concentration by weight to isopropyl myristate and dissolving the lecithin in the isopropyl myristate with constant stirring at room temperature.

A polar phase was prepared by dispersing NOVAXAN D brand xanthan gum, a water dispersible transparent xanthan gum, available from Archer-Daniels-Midland Company of Decatur, IL, at 0.6-1.0% in distilled water at room temperature.

The polar phase was slowly introduced into the organic phase under constant stirring at a concentration of 4% at room temperature. At this point, the lecithin organic phase spontaneously changed from a Newtonian fluid to a viscous gel phase, also referred to as the lecithin organogel. Upon heating, the lecithin organogel became fluid and self-assembled back into the lecithin organogel upon cooling, indicating the thermo-reversible property of the lecithin organogel.

### Example 5.

An organic phase was prepared by adding alcohol fractionated lecithin (approximately 40% phosphatidyl choline) at 85% concentration by weight to isopropyl myristate at 11% by weight concentration, and dissolving the lecithin in the isopropyl myristate with constant stirring at room temperature.

A polar phase was prepared by dispersing NOVAXAN D brand xanthan gum, a water dispersible transparent xanthan gum, available from Archer-Daniels-Midland Company of Decatur, IL, at 0.6-1.0% in distilled water at room temperature.

The polar phase was slowly introduced into the organic phase under constant stirring at a concentration of 4% at room temperature. At this point, the lecithin organic phase spontaneously changed from a Newtonian fluid to a viscous gel phase, also referred to as the lecithin organogel. Upon heating, the lecithin organogel became fluid and self-assembled back into the lecithin organogel upon cooling, indicating the thermo-reversible property of the lecithin organogel.

### Example 6.

An organic phase was prepared by adding PHOSPHOLIPON 90 brand lecithin (approximately 90% phosphatidyl choline), a high purity lecithin available from American Lecithin Company, Oxford, CT, at 85% concentration by weight to isopropyl myristate at 11% by weight concentration, and dissolving the lecithin in the isopropyl myristate with constant stirring at room temperature.

A polar phase was prepared by dispersing NOVAXAN D brand xanthan gum, a water dispersible transparent xanthan gum, available from Archer-Daniels-Midland Company of Decatur, IL, at 0.6-1.0% in distilled water at room temperature.

The polar phase was slowly introduced into the organic phase under constant stirring at a concentration of 4% at room temperature. At this point, the lecithin organic phase spontaneously changed from a Newtonian fluid to a viscous gel phase, also referred to as the lecithin organogel. Upon heating, the lecithin organogel became fluid and self-assembled back into the lecithin organogel upon cooling, indicating the thermo-reversible property of the lecithin organogel.

### Example 7.

An organic phase was prepared by adding YELKIN SS brand lecithin, a standardized fluid lecithin, available from Archer-Daniels-Midland Company of Decatur, IL, at 80% concentration by weight to isopropyl myristate and dissolving the lecithin in the isopropyl myristate with constant stirring at room temperature. Vitamin E, available from Archer-Daniels-Midland Company of Decatur, IL, at a concentration of 2% was added to this organic phase and stirred.

A polar phase was prepared by dispersing NOVAXAN D brand xanthan gum, a water dispersible transparent xanthan gum, available from Archer-Daniels-Midland Company of Decatur, IL, at 0.6-1.0% in distilled water at room temperature. GUARDIAN brand green tea extract, available from Danisco USA Inc., New Century, KS, was added to the polar phase at a concentration of 2%.

The polar phase was slowly introduced into the organic phase under constant stirring at a concentration of 4% at room temperature. At this point, the lecithin organic phase spontaneously changed from a Newtonian fluid to a viscous gel phase, also referred to as the lecithin organogel. Upon heating, the lecithin organogel became fluid and self-assembled back into the lecithin organogel upon cooling, indicating the thermo-reversible property of the lecithin organogel.

### Example 8.

An organic phase was prepared by adding YELKIN SS brand lecithin, available from Archer-Daniels-Midland Company of Decatur, IL, at 80% concentration by weight to isopropyl myristate and dissolving the lecithin in the isopropyl myristate with constant stirring at room temperature. CARDIOAID brand phytosterols, available from Archer-Daniels-Midland Company of Decatur, IL, at a concentration of 2% was added to this organic phase, heated and stirred to dissolve the solids. Once the solids were dissolved, the organic phase was allowed to cool to room temperature.

A polar phase was prepared by dispersing NOVAXAN D brand xanthan gum, a water dispersible and transparent xanthan gum, available from Archer-Daniels-Midland Company of Decatur, IL, at 0.6-1.0% in distilled water at room temperature. GUARDIAN brand green tea extract, available from Danisco USA Inc., New Century, KS, was added to the polar phase at a concentration of 2%.

The polar phase was slowly introduced into the organic phase under constant stirring at a concentration of 4% at room temperature. At this point, the lecithin organic phase spontaneously changed from a Newtonian fluid to a viscous gel phase, also referred to as the lecithin organogel. Upon heating, the lecithin organogel became fluid and self-assembled back into the lecithin organogel upon cooling, indicating the thermo-reversible property of the lecithin organogel.

### Example 9.

An organic phase was prepared by dispersing about 5 grams of ULTRALEC P brand de-oiled lecithin, available from Archer-Daniels-Midland Company of Decatur, IL, in isopropyl palmitate under high shear.

A polar phase was prepared by dispersing NOVAXAN 80 brand transparent xanthan gum, a water dispersible xanthan gum, available from Archer-Daniels-Midland Company of Decatur, IL, at 2% in water at room temperature, thus producing a transparent gel.

The organic phase was incorporated into the polar phase with gentle mixing, thus preparing the xanthan-lecithin organogel.

### Example 10.

An organic phase was prepared by adding YELKIN SS brand lecithin, available from Archer-Daniels-Midland Company, Decatur, IL, at 80% concentration by weight to isopropyl palmitate. The lecithin was dissolved in the isopropyl palmitate with constant stirring at room temperature.

A polar phase was prepared by dispersing NOVAXAN D brand xanthan gum, a water dispersible transparent xanthan gum available from Archer-Daniels-Midland Company, Decatur, IL, at 0.75% (w/v) along with ULTRALEC P, a water dispersible powdered lecithin available from Archer-Daniels-Midland Company, Decatur, IL, at 1% (w/v), and 0.5% potassium sorbate in distilled water at room temperature.

The polar phase was slowly introduced into the organic phase under constant stirring at concentrations of 4-25% (w/v) at room temperature. The lecithin organic phase spontaneously changed from a Newtonian fluid to a viscous gel phase, the lecithin organogel. Upon heating, the lecithin organogel became fluid and self-assembled back into the lecithin organogel upon cooling, indicating the thermo-reversible property of the lecithin organogel.

### Example 11.

An organic phase was prepared by adding YELKIN SS brand lecithin, available from Archer-Daniels-Midland Company, Decatur, IL, at 70% concentration by weight to 10% (w/v) isopropyl palmitate and 10% (w/v) diglyceride oil available from Kao Corporation. The lecithin was dissolved in the mixture of isopropyl palmitate and diglyceride oil with constant stirring at room temperature to form the organic phase.

A polar phase was prepared by dispersing NOVAXAN D brand xanthan gum, a water dispersible transparent xanthan gum available from Archer-Daniels-Midland Company, Decatur, IL, at 0.75% (w/v) along with ULTRALEC P brand lecithin, a water dispersible powdered lecithin available from Archer-Daniels-Midland Company, Decatur, IL, at 1% (w/v) and 0.5% potassium sorbate in distilled water at room temperature.

The polar phase was slowly introduced into the organic phase under constant stirring at concentrations of 10% (w/v) at room temperature. The lecithin organic phase spontaneously changed from a Newtonian fluid to a viscous gel phase, the lecithin organogel. Upon heating, the lecithin organogel became fluid and self assembled back into the lecithin organogel upon cooling, indicating the thermo-reversible property of the lecithin organogel.

### Example 12.

An organic phase was prepared by adding YELKIN SS brand lecithin, available from Archer-Daniels-Midland Company, Decatur, IL, at 70% concentration by weight to 10% (w/v) isopropyl palmitate and 10% (w/v) high oleic sunflower oil. The lecithin was dissolved in the mixture of isopropyl palmitate and high oleic sunflower oil with constant stirring at room temperature.

A polar phase was prepared by dispersing NOVAXAN D brand xanthan gum, a water dispersible transparent xanthan gum available from Archer-Daniels-Midland Company, Decatur, IL, at 0.75% (w/v) with ULTRALEC P brand lecithin, a water dispersible powdered lecithin available from Archer-Daniels-Midland Company of Decatur, IL, and 0.5% potassium sorbate in distilled water at room temperature.

The polar phase was slowly introduced into the organic phase under constant stirring at concentrations of 10% (w/v) at room temperature. The lecithin organic phase spontaneously changed from a Newtonian fluid to a viscous gel phase, the lecithin organogel. Upon heating, the lecithin organogel became fluid and self assembled back into the lecithin organogel upon cooling, indicating the thermo-reversible property of the lecithin organogel.

### Example 13.

An organic phase was prepared by adding YELKIN SS brand lecithin, available from Archer-Daniels-Midland Company, Decatur, IL, at 70% concentration by weight to 20% (w/v) diglyceride oil available from Kao Corporation and dissolved the lecithin in the diglyceride oil with constant stirring at room temperature.

A polar phase was prepared by dispersing NOVAXAN D brand xanthan gum, a water dispersible transparent xanthan gum available from Archer-Daniels-Midland Company, Decatur, IL, at 0.75% (w/v) with ULTRALEC P, a water dispersible powdered lecithin available from Archer-Daniels-Midland Company, Decatur, IL, at 1% (w/v) and 0.5% potassium sorbate at room temperature.

The polar phase was slowly introduced into the organic phase under constant stirring at a concentration of 10% (w/v) at room temperature. The lecithin organic phase spontaneously changed from a Newtonian fluid to a viscous gel phase, also referred to as the lecithin organogel. Upon heating, the lecithin organogel became fluid and self assembled back into the lecithin organogel upon cooling, indicating the thermo-reversible property of the lecithin organogel.

### Example 14.

An organic phase was prepared by adding YELKIN SS brand lecithin, available from Archer-Daniels-Midland Company, Decatur, IL, at 75% concentration by weight to 20% (w/v) of PGE 3-4-0, a polyglyercol ester, (Polyaldo 3-4-0, available from Lonza Group Ltd., Basel, Switzerland) and dissolving the lecithin in PGE 3-4-0 with constant stirring at room temperature.

A polar phase was prepared by dispersing NOVAXAN D brand xanthan gum, a water dispersible transparent xanthan gum available from Archer-Daniels-Midland Company, Decatur, IL, at 0.75% (w/v) and ULTRALEC P brand lecithin, a water dispersible powdered lecithin available from Archer-Daniels-Midland Company, Decatur, IL, and 0.5% potassium sorbate in distilled water at room temperature.

The polar phase was slowly introduced into the organic phase under constant stirring at a concentration of 5% (w/v) at room temperature. The lecithin organic phase spontaneously changed from a Newtonian fluid to a viscous gel phase, the lecithin organogel. Upon heating, the lecithin organogel became fluid and self-assembled back into the lecithin organogel upon cooling, indicating the thermo-reversible property of the lecithin organogel.

### Example 15.

An organic phase was prepared by adding YELKIN SS brand lecithin, available from Archer-Daniels-Midland Company, Decatur, IL, at 75% concentration by weight to 10% (w/v) of PGE 3-4-0, a polyglyercol ester, (Polyaldo 3-4-0, available from Lonza Group Ltd., Basel, Switzerland) and 10% (w/v) diglyceride oil available from Kao Corporation and dissolving the lecithin in the mixture of the PGE 3-4-0 and the diglyceride oil with constant stirring at room temperature.

A polar phase was prepared by dispersing NOVAXAN D brand xanthan gum, a water dispersible transparent xanthan gum available from Archer-Daniels-Midland Company, Decatur, IL, at 0.75% (w/v) along with ULTRALEC P brand lecithin, a water dispersible powdered lecithin available from Archer-Daniels-Midland Company, Decatur, IL, and 0.5% potassium sorbate as a preservative in distilled water at room temperature.

The polar phase was slowly introduced into the organic phase under constant stirring at concentrations of 5% (w/v) at room temperature. The lecithin organic phase spontaneously changed from a Newtonian fluid to a viscous gel phase, the lecithin organogel. Upon heating, the lecithin organogel became fluid and self assembled back into the lecithin organogel upon cooling, indicating the thermo-reversible property of the lecithin organogel.

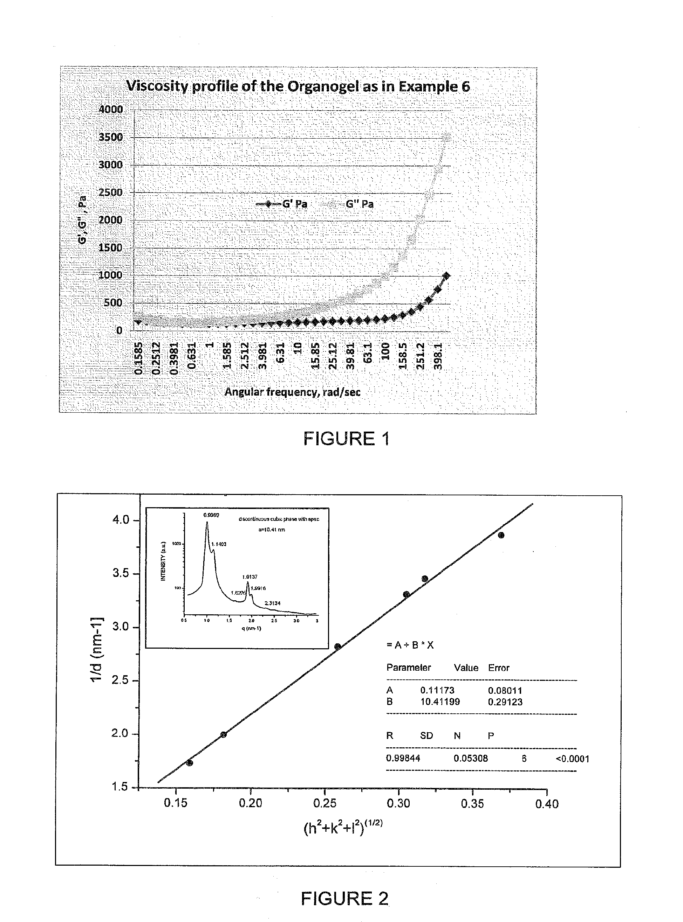

Rheology measurements were performed on an AR-2000 Stress Controlled Rheometer (TA), with cone/plate geometry (2° angle and 40 mm diameter; gap 51 mm). The oscillation frequency sweep was carried out at 25°C at 12% strain in the angular frequency range of 0.1 to 500 rad/sec. FIG. 1 shows the storage modulus (G') and loss modulus (G") plotted against the angular frequency (rad/sec). The G", loss modulus, was always higher than G', storage modulus, indicating a more viscous behavior of the gel over the entire frequency range studied.

Polarized light microscopy (PLM) can be used to determine whether the composition formed a cubic phase. The colloidal phase can be defined from the textures obtained in the microscope. Unlike the anisotropic phase structures (lamellar and hexagonal), cubic phases showed no birefringence and appeared dark in the microscope.

The cubic phase is also confirmed by Small Angle X-ray Scattering (SAXS). Studies were performed at the Basali Institute of Applied Chemistry, The Hebrew University of Jerusalem, Israel, to identify the structure and the degree of internal order of the bulk liquid crystalline phases. In FIG. 2, SAXS scattering curves are shown. 0.999, 1.1403, 1.6205, 1.9137, 1.9916 and 2.3134 nm that have been translated into spacing ratios of √3,√4,√8,√11,√12 and √15 The plot of the reciprocal spacing, 1/d _{h, k, l} versus the (h² + k²+ l²)^{1/2} value of all six of the diffraction peaks exhibit linearity with R=0.9984. The indexing space can be interpreted for Fm3m space group of cubic symmetry with a lattice parameter of 104A. This value was similar to the one derived for the monoolein-water-ethanol cubic bicontinuous phase as determined in R.Efrat, A.Aserin, E.Kesselman, D. Danino, E.Wachtel and N.Garti, Colloids and Surfaces A: Physicochem. Eng. Aspects 299 (2007), 133-145.

### Example 16.

An organic phase was prepared by adding YELKIN SS brand lecithin, available from Archer-Daniels-Midland Company, Decatur, IL, at 65% concentration by weight to 10% (w/v) isopropyl palmitate and 10% (w/v) diglyceride oil available from Kao Corporation and dissolving the lecithin in the mixture of isopropyl palmitate and diglyceride oil with constant stirring at room temperature. While stirring, 6 grams of vitamin E and 6 grams of glycerol were added.

A polar phase was prepared by dispersing NOVAXAN D brand xanthan gum, a water dispersible transparent xanthan gum available from Archer-Daniels-Midland Company, Decatur, IL, at 0.75% (w/v) along with ULTRALEC P, a water dispersible powdered lecithin available from Archer-Daniels-Midland Company, Decatur, IL, at 1%(w/v) and 0.5% potassium sorbate in distilled water at room temperature.

The polar phase was slowly introduced into the organic phase under constant stirring at concentrations of 15% (w/v) at room temperature. The lecithin organic phase spontaneously changed from a Newtonian fluid to a viscous gel phase, referred to as the lecithin organogel. Upon heating, the lecithin organogel became fluid and self assembled back in to the lecithin organogel upon cooling, indicating the thermo-reversible property of the lecithin organogel.

### Example 17.

An organic phase was prepared by adding YELKIN SS brand lecithin, available from Archer-Daniels-Midland Company, Decatur, IL, at 70% concentration by weight to 10% (w/v) PGE 3-4-0, a polyglyercol ester, (Polyaldo 3-4-0, available from Lonza Group Ltd., Basel, Switzerland), 10% (w/v) high oleic sunflower oil and 5 grams of monoglyceride (Dimodan SO/D K-A, available from Danisco, New Century, KS). The lecithin was dissolved in the mixture of PGE 3-4-0, high oleic sunflower oil and the monoglyceride with constant stirring at room temperature.

A polar phase was prepared by dispersing NOVAXAN D brand xanthan gum, a water dispersible transparent xanthan gum available from Archer-Daniels-Midland Company, Decatur, IL, at 0.75% (w/v) along with ULTRALEC P, a water dispersible powdered lecithin available from Archer-Daniels-Midland Company, Decatur, IL, at 1% (w/v), and 0.5% potassium sorbate in distilled water at room temperature.

The polar phase was slowly introduced into the organic phase under constant stirring at concentrations of 10% (w/v) at room temperature. The lecithin organic phase spontaneously changed from a Newtonian fluid to a viscous gel phase, referred to as the lecithin organogel. Upon heating, the lecithin organogel became fluid and self assembled back in to the lecithin organogel upon cooling, indicating the thermo-reversible property of the lecithin organogel.

### Example 18.

An organic phase was prepared by adding YELKIN SS brand lecithin, available from Archer-Daniels-Midland Company, Decatur, IL, at 75% concentration by weight to 10% (w/v) of PGE 3-4-0, a polyglyercol ester, (Polyaldo 3-4-0, available from Lonza Group Ltd., Basel, Switzerland), 10% (w/v) high oleic sunflower oil and 5 grams of monoglyceride (Dimodan SO/D K-A, available from Danisco, New Century, KS). The lecithin was dissolved in the mixture of PGE 3-4-0, high oleic sunflower oil and the monoglyceride with constant stirring at room temperature. Upon stirring, 6% of glycerol was added to the mixture.

A polar phase was prepared by dispersing NOVAXAN D brand xanthan gum, a water dispersible transparent xanthan gum available from Archer-Daniels-Midland Company, Decatur, IL, at 0.75% (w/v) along with ULTRALEC P brand lecithin, a water dispersible powdered lecithin available from Archer-Daniels-Midland Company, Decatur, IL, at 1% (w/v), and 0.5% potassium sorbate in distilled water at room temperature.

The polar phase was slowly introduced into the organic phase under constant stirring at concentrations of 5% (w/v) at room temperature. The lecithin organic phase spontaneously changed from a Newtonian fluid to a viscous gel phase, referred to as the lecithin organogel. Upon heating, the lecithin organogel became fluid and self assembled back in to the lecithin organogel upon cooling, indicating the thermo-reversible property of the lecithin organogel.

### Example 19.

An organic phase was prepared by adding YELKIN SS brand lecithin, available from Archer-Daniels-Midland Company, Decatur, IL, at 67% concentration by weight to 8.4% (w/v) PGE 3-4-0, a polyglyercol ester, (Polyaldo 3-4-0, available from Lonza Group Ltd., Basel, Switzerland), 17.6% (w/v) high oleic sunflower oil and 10 grams of CARDIOAID brand sterols available from Archer-Daniels-Midland Company, Decatur, IL, was added into 26 grams of the oil phase. The lecithin was dissolved in the mixture of PGE 3-4-0, and high oleic sunflower oil along with the CARDIOAID brand sterols under constant stirring at room temperature.

A polar phase was prepared by dispersing NOVAXAN D brand xanthan gum, a water dispersible transparent xanthan gum available from Archer-Daniels-Midland Company, Decatur, IL, at 0.75% (w/v) along with ULTRALEC P brand lecithin, a water dispersible powdered lecithin available from Archer-Daniels-Midland Company, Decatur, IL, at 1% (w/v), and 0.5% potassium sorbate in distilled water at room temperature.

The polar phase was slowly introduced into the organic phase under constant stirring at concentrations of 7% (w/v) at room temperature. The lecithin organic phase spontaneously changed from a Newtonian fluid to a viscous gel phase, referred to as the lecithin organogel. Upon heating, the lecithin organogel became fluid and self assembled back in to the lecithin organogel upon cooling, indicating the thermo-reversible property of the lecithin organogel.

### Example 20.

The edible version of the organogel having the polyglycerol ester and/or vegetable oil is blended with high oleic sunflower oil at a ratio of 10-40%. The resulting mixture is heated to 40-50°C in order to have a clear, transparent liquid of oil like consistency which on cooling forms a film on a substrate. This could be used, *inter alia,* as a sprayable oil as a carrier of spices, flavors and/or colorings for snack food applications including, but not limited to, chips.

### Example 21.

The organogels prepared herein are all thermo-reversible. Taking advantage of the thermo-reversible nature of these gels, the loading of bioactive substances was carried out after making the lecithin organogel. A lecithin organogel was prepared as described in Example 6.

This lecithin organogel was heated to 40°C to completely melt and under constant stirring, NOVATOL 6-92 brand vitamin E, a non-polar antioxidant available from Archer-Daniels-Midland Company of Decatur, IL, was slowly introduced in the molten lecithin organogel, followed by the gradual addition of green tea extract at a 15% concentration in USP grade glycerol. The molten lecithin organogel was cooled to room temperature and the lecithin organogel was reformed partitioning the vitamin E and the polar phase, having the green tea extract in glycerol, in the respective phases without changing the nature of the lecithin organogel. The thermo-reversible nature of the lecithin organogel including the vitamin E and green tea extract was confirmed by viscosity measurements before and after the vitamin E and green tea extract were added at different concentrations.

The rheology measurements were performed on an AR-2000 Stress Controlled Rheometer (TA), with cone/plate geometry (2° angle and 40 mm diameter; gap 51 mm). The oscillation frequency sweep was carried out at 25°C at 12 % strain in the angular frequency range of 0.1 to 500 rad/sec.

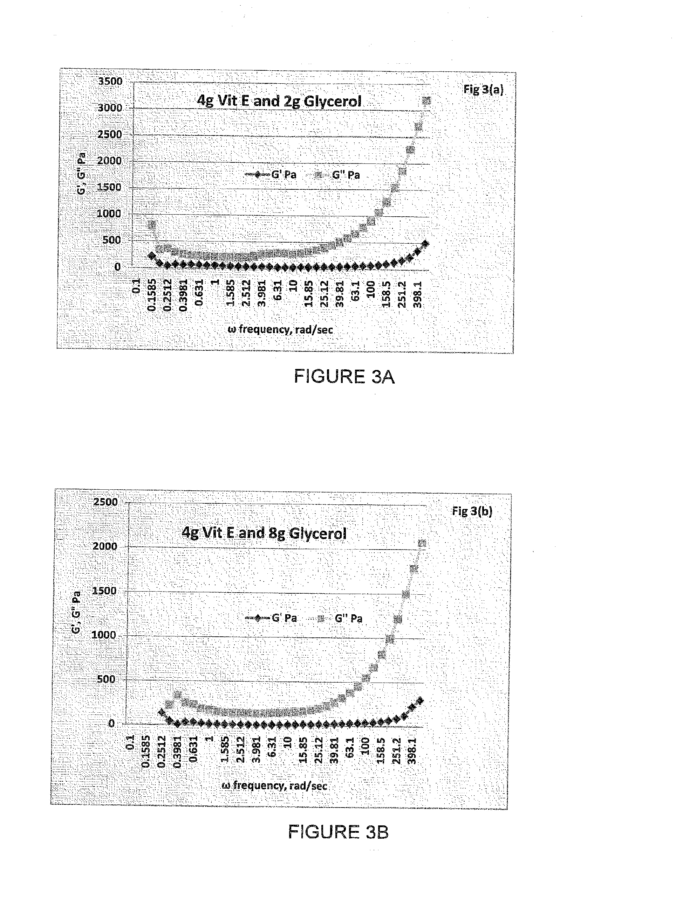

The viscosity profile remained constant as shown in FIG. 3A and FIG. 3B. FIG. 3A and FIG. 3B show the storage modulus (G') and loss modulus (G") plotted against the angular frequency (rad/s). The G", loss modulus, was always higher than G', storage modulus, indicating a more viscous behavior of the lecithin organogel over the entire frequency range studied.

This property makes the lecithin organogel of the present invention unique as any desired active substances can be added to the lecithin organogel anytime after the lecithin organogels are prepared.

Polarized light microscopy (PLM) can be used to determine whether the composition formed a cubic phase. The colloidal phase can be defined from the textures obtained in the microscope. Unlike the anisotropic phase structures (lamellar and hexagonal), cubic phases showed no birefringence and appeared dark in the microscope.

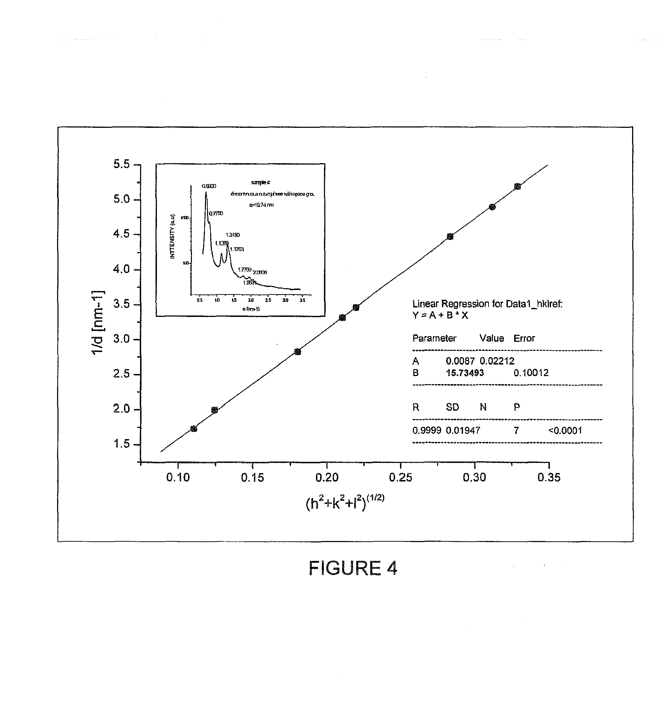

The cubic phase is also confirmed by Small Angle X-ray Scattering (SAXS). Studies were performed to identify the structure and the degree of internal order of the bulk liquid crystalline phases. In FIG. 4, SAXS scattering curves are shown with 8 major peaks at 0.692, 0.7783, 1.1288, 1.318, 1.3763, 1.7759, 1.9531 and 2.0606 nm that have been translated into spacing ratios of √3,√4,√8,√11,√12,√20 and √27, Plot of the reciprocal spacing 1/d _{h, k, l} versus the (h² + k²+ l²)^{1/2} value of all the six diffraction peaks exhibit linearity with R=0.9999. The indexing space can be interpreted for Fm3m space group of cubic symmetry with a lattice parameter of 157A. This value was similar to the one derived for the GMO-water mixtures for the existence of cubic bicontinuous phase with lattice parameter of 130A. This could be the effect of the added glycerol and the bigger molecule of vitamin E acetate incorporated in the cubic phase as discussed in R.Efrat, A.Aserin, E.Kesselman, D. Danino, E.Wachtel and N.Garti, Colloids and Surfaces A: Physicochem. Eng. Aspects 299 (2007), 133-145.

The present invention has been described with reference to certain exemplary embodiments, compositions and uses thereof. However, it will be recognized by those of ordinary skill in the art that various substitutions, modifications or combinations of any of the exemplary embodiments may be made without departing from the spirit and scope of the invention. Thus, the invention is not limited by the description of the exemplary embodiment, but rather by the appended claims as originally filed.

## Claims

1. A thermo-reversible, structured phospholipid organogel composition comprising:
a phospholipid composition;
an organic solvent,
in particular selected from the group consisting of isopropyl myristate, ethyl laureate, ethyl myristate, isopropyl palmitate, cyclopentane, cyclooctane, trans-decalin, trans-pinane, n-pentane, n-hexane, n-hexadecane, tripropylamine, 1 ,7-octadiene, butyl laurate, cyclododecane, dibutyl ether, isooctane, n- octane, tributylamine, triisobutylamine, mineral oil, vegetable oil such as triglyceride and/or diglyceride oils, a polyol esters, monoglycerides, diglycerides, fatty acid esters and combinations of any thereof;
a water soluble polymer,
in particular a bio-based polymer such as a bio-based polymer as determined by ASTM International Radioisotope Standard Method D 6866; and
a polar solvent,
in particular selected from the group consisting of water, glycerol, ethylene glycol, propylene glycol, formamide, isosorbide, isosorbide derivatives, sorbitol, erythritol, other polyhydric alcohols and combinations of any thereof.

2. The thermo-reversible, structured phospholipid organogel composition of claim 1, wherein upon heating of the thermo-reversible, structured phospholipid organogel to a temperature between 30-40°C, the thermo-reversible, structured phospholipid organogel composition melts and wherein upon cooling the melted thermo- reversible, structured phospholipid organogel composition to a temperature of below 30°C, the thermo-reversible, structured phospholipid organogel composition reforms to the shape of a gel.

3. The thermo-reversible, structured phospholipid organogel composition of any one of claims 1 or 2, further comprising a compound selected from the group consisting of green tea extract, a fragrance, ascorbic acid, potassium sorbate, citric acid, natural polar antioxidants, tocopherols, sterols, phytosterols, saw palmetto, caffeine, sea weed extract, grape-seed extract, rosemary extract, almond oil, lavender oil, peppermint oil, bromelain, capsaicin, benzalkaonium chloride, triclosan, para-chloro-meta xylenol (PCMX), hyalauronic acid, emulsifiers, a polar guest molecule, a non-polar guest molecule, an amphilic guest molecules, an enzyme and combinations of any thereof.

4. The thermo-reversible, structured phospholipid organogel composition of any one of claims 1-3, further comprising a compound selected from the group consisting of an anesthetic, a nonsteroidal anti-inflammatory drug, a muscle relaxant, a steroid, a hormone, an analgesic, an antiemetic, a cardiovascular agent, an antithyroid drug, a macromolecule, a neuropathy drug, a sanitizer, a disinfectant and combinations of any thereof.

5. The thermo-reversible, structured phospholipid organogel composition of any one of claims 1-4, wherein the phospholipid composition comprises less than 90% phosphatides, less than 30% phosphatidyl choline or between 10-95% phosphatidyl choline.

6. The thermo-reversible, structured phospholipid organogel composition of any one of claims 1-5, wherein the water soluble polymer is selected from the group consisting of xanthan gum, gellan gum, cellulose and modified cellulose products, starch, chitin, carrageenan, gum arabic, an alginate, gum acacia, guar gum, agar, gelatin, locus bean gum, inulin, maltodextrin, pectin, beta glucans, and combinations of any thereof.

7. The thermo-reversible, structured phospholipid organogel composition of any one of claims 1-6, wherein the water soluble polymer is mixed with de-oiled lecithin.

8. Use of the thermo-reversible, structured phospholipid organogel composition of any one of claims 1-7 in a food product, a cosmetic, a personal care product, or an industrial product.

9. A process for producing a product, the process comprising: mixing a organic solvent with a phospholipid composition, thus producing an organic phase; dispersing a water soluble polymer in a polar solvent, thus producing a polar phase; and mixing the organic phase with the polar phase.

10. The process of claim 9, further comprising adding a compound selected from the group consisting of green tea extract, a fragrance, ascorbic acid, potassium sorbate, citric acid, natural polar antioxidants, tocopherols, sterols, phytosterols, saw palmetto, caffeine, sea weed extract, grape-seed extract, rosemary extract, almond oil, lavender oil, peppermint oil, bromelain, capsaicin, benzalkaonium chloride, triclosan, para- chloro-meta xylenol (PCMX), hyalauronic acid, emulsifiers, a polar guest molecule, a non- polar guest molecule, an amphilic guest molecules, an enzyme and combinations of any thereof to the organic phase, the polar phase or a combination thereof.

11. The process of claim 9 or claim 10, wherein the organic solvent and the phospholipid composition are mixed under constant stirring and/or wherein the water soluble polymer is dispersed in the polar solvent under constant stirring.

12. The process of any one of claims 9-11, wherein the process takes place at ambient temperature and low shear.

13. A method of loading thermo-reversible, structured phospholipid organogel, the method comprising: melting the thermo-reversible, structured phospholipid organogel; mixing a compound, which is in particular
selected from the group consisting of a hydrophobic compound, a hydrophilic compound, an amphiphilic compound, and combinations of any thereof,
with the melted thermo-reversible, structured phospholipid organogel; and cooling the thermo-reversible, structured phospholipid organogel including the compound to a temperature below the melting point such that the thermo-reversible, structured phospholipid organogel reforms to the shape of a gel.

14. The method of claim 13, further comprising incorporating the thermo-reversible, structured phospholipid organogel into a food product, a cosmetic, a personal care product, or an industrial product.

15. A product obtainable by the method of claim 13 or 14.
